# EUROPÄISCHE PATENTSCHRIFT

(11) **EP 4 136 353 B1**
(45) Veröffentlichungstag und Bekanntmachung des Hinweises auf die Patenterteilung: **06.03.2024**
(21) Anmeldenummer: 21806184.4
(22) Anmeldetag: 03.11.2021
(51) Int. Cl.: F04B 9/14, F04B 43/12, F16K 31/05

(54) **PERISTALTIKPUMPE FÜR EINE VORRICHTUNG ZUR EXTRAKORPORALEN BLUTBEHANDLUNG**
PERISTALTIC PUMP FOR A DEVICE FOR EXTRACORPOREAL BLOOD TREATMENT
POMPE PÉRISTALTIQUE POUR UN DISPOSITIF DE TRAITEMENT EXTRACORPOREL DU SANG

(30) Priorität: 06.11.2020 DE 102020213988
(43) Veröffentlichungstag der Anmeldung: 22.02.2023
(73) Patentinhaber: B. Braun Avitum AG, 34212 Melsungen (DE)
(72) Erfinder: JANIK, Waldemar, 34212 Melsungen (DE)
(74) Vertreter: Patentanwälte Ruff, Wilhelm, Beier, Dauster & Partner mbB
(86) Internationale Anmeldenummer: PCT/EP2021/080507
(87) Internationale Veröffentlichungsnummer: WO 2022/096502

(56) Entgegenhaltungen:
- EP-A2- 1 457 677
- CN-A- 107 842 622
- CN-A- 108 661 872
- CN-A- 111 336 302
- US-A- 2 679 807
- US-A- 4 759 386
- US-A1- 2005 095 155

## Beschreibung

Die Erfindung betrifft eine Peristaltikpumpe für eine Vorrichtung zur extrakorporalen Blutbehandlung, aufweisend einen um eine Rotorachse rotierbaren Rotor mit einem Rotorgrundkörper und ein Pumpengehäuse mit einer Aufnahmeaussparung, in welcher der Rotor aufgenommen ist, und welche eine bogenförmig um die Rotorachse erstreckte und radial von dem Rotor beabstandete Stützfläche aufweist, die zum Abstützen eines radial zwischen den Rotor und die Stützfläche einbringbaren Schlauchsegments eingerichtet ist, wobei der Rotor wenigstens ein erstes Betätigungselement aufweist, welches zur manuellen Drehbetätigung des Rotors um die Rotorachse eingerichtet ist, und welches wenigstens einen ersten Betätigungsabschnitt zum Aufbringen einer manuellen Drehbetätigungskraft aufweist.

Eine derartige Peristaltikpumpe ist aus der US 2004/0179964 A1 bekannt und zur Förderung von Blut durch einen extrakorporalen Blutkreislauf eines Dialysegeräts vorgesehen. Die bekannte Peristaltikpumpe weist ein Pumpengehäuse mit einer bogenförmig erstreckten Stützfläche und einen innerhalb der Stützfläche um eine Rotorachse rotierbaren Rotor mit einem Rotorgrundkörper auf. Zwischen die Stützfläche und den Rotor ist ein Schlauchsegment einbringbar. Zur Pumpförderung des Bluts wird das Schlauchsegment unter Einwirkung des rotierenden Rotors lokal eingedrückt. Dabei wird der Rotor motorisch angetrieben. Der Rotor weist zudem ein fest an dem Rotorgrundkörper gelagertes Betätigungselement in Form eines Drehknebels auf. Zum manuellen Notbetrieb der Peristaltikpumpe kann der Rotor mittels einer manuellen Drehbetätigung des Drehknebels um die Rotorachse rotiert werden.

Aus der DE 10 2012 105 913 A1 ist eine weitere Peristaltikpumpe bekannt. Zum manuellen Notbetrieb ist ein schwenkbeweglich an dem Rotorgrundkörper gelagerter Kurbelgriff vorgesehen. Dieser ist zwischen einer Ruhestellung und einer Betätigungsstellung verlagerbar. In der Ruhestellung ist der Kurbelgriff manuell unzugänglich in einer Aussparung des Rotorgrundkörpers aufgenommen, so dass eine manuelle Drehbetätigung unmöglich ist.

Das Dokument EP 1 457 677 offenbart eine Peristaltikpumpe, welche von Hand betrieben werden kann.

Aufgabe der Erfindung ist es, eine Peristaltikpumpe der eingangs genannten Art bereitzustellen die einen zuverlässigen Notbetrieb gewährleistet und gleichzeitig einen möglichst einfachen Aufbau aufweist.

Diese Aufgabe wird dadurch gelöst, dass das erste Betätigungselement wenigstens mittelbar an dem Rotorgrundkörper gelagert und relativ zu dem Rotorgrundkörper wenigstens abschnittsweise beweglich ist zwischen einer ersten Betätigungsstellung, in welcher der erste Betätigungsabschnitt um eine erste Hebelarmlänge von der Rotorachse beabstandet ist, und einer zweiten Betätigungsstellung, in welcher der erste Betätigungsabschnitt um eine zweite Hebelarmlänge von der Rotorachse beabstandet ist, wobei die zweite Hebelarmlänge größer ist als die erste Hebelarmlänge. Durch die erfindungsgemäße Lösung kann das erste Betätigungselement situationsangepasst zwischen unterschiedlichen Betätigungsstellungen, nämlich der ersten und der zweiten Betätigungsstellung, verlagert werden. Dabei geht die Erfindung von der Erkenntnis aus, dass je nach Situation ein vergleichsweise größeres oder kleineres manuelles Antriebsdrehmoment zum Rotieren des Rotors erforderlich und/oder wünschenswert sein kann. Durch die wenigstens abschnittsweise Beweglichkeit des ersten Betätigungselements kann die um die Rotorachse wirksame Hebelarmlänge des ersten Betätigungsabschnitts auf einfache Weise verändert werden. Dies ermöglicht - unter Beibehaltung ein- und derselben manuellen Drehbetätigungskraft - eine einfache Anpassung des resultierenden manuellen Antriebsdrehmoments. Sofern eine die Peristaltikpumpe bedienende Person beispielsweise feststellen sollte, dass sich der Rotor in der ersten Betätigungsstellung nicht oder lediglich schwergängig manuell rotieren lässt, kann eine Verlagerung in die zweite Betätigungsstellung erfolgen. Durch die dann wirksame vergleichsweise größere zweite Hebelarmlänge kann der Rotor dementsprechend leichtgängiger manuell rotiert werden. Letztlich wird hierdurch ein besonders zuverlässiger Notbetrieb der Peristaltikpumpe ermöglicht. Zur Verlagerung des ersten Betätigungsabschnitts zwischen der ersten und der zweiten Betätigungsstellung ist das erste Betätigungselement wenigstens abschnittsweise relativ zu dem Rotorgrundkörper beweglich. Beispielsweise kann lediglich der erste Betätigungsabschnitt relativ zu dem Rotorgrundkörper beweglich sein, wobei weitere Abschnitte des ersten Betätigungselements relativ zu dem Rotorgrundkörper unbeweglich an demselben gelagert sind. Alternativ kann das gesamte erste Betätigungselement relativ zu dem Rotorgrundkörper beweglich an demselben gelagert sein. Der erste Betätigungsabschnitt und/oder das erste Betätigungselement ist vorzugsweise linearbeweglich zwischen der ersten und der zweiten Betätigungsstellung verlagerbar. Vorzugsweise weist der Rotor zu diesem Zweck eine Linearführung auf. Alternativ kann das erste Betätigungselement teleskopierbar gestaltet sein. Weiter alternativ kann eine schwenkbewegliche Verlagerung des ersten Betätigungselements mittels einer Schwenkführung oder dergleichen vorgesehen sein. Das erste Betätigungselement kann mittelbar oder unmittelbar an dem Rotorgrundkörper gelagert sein. In der ersten Betätigungsstellung ist der erste Betätigungsabschnitt in Bezug auf die Rotorachse radial innenliegend angeordnet. In der zweiten Betätigungsstellung ist der erste Betätigungsabschnitt in Bezug auf die Rotorachse radial außenliegend angeordnet. Vorzugsweise bilden die erste und die zweite Betätigungsstellung jeweils eine Endlage entlang des Verlagerungsweges, so dass auch von einer ersten Endlage und einer zweiten Endlage gesprochen werden kann. Die besagten Endlagen oder auch -stellungen sind von etwaigen Zwischenstellungen des ersten Betätigungsabschnitts und/oder ersten Betätigungselements zu unterscheiden. In solchen Zwischenstellungen liegt eine konstruktiv festgelegte, wenigstens einseitig begrenzte und/oder stabile Relativposition gegenüber dem Rotorgrundkörper nicht vor. Mit anderen Worten ausgedrückt, nimmt der erste Betätigungsabschnitt und/oder das erste Betätigungselement in der ersten Betätigungsstellung vorzugsweise eine entlang des Verlagerungswegs konstruktiv festgelegte, wenigstens einseitig begrenzte und/oder stabile Relativposition gegenüber dem Rotorgrundkörper ein. Die besagte wenigstens einseitige Begrenzung kann insbesondere form- und/oder kraftschlüssig, mittels eines Anschlags, einer Rastung oder dergleichen bewirkt sein. Vorzugsweise gilt dies sinngemäß entsprechend für die zweite Betätigungsstellung. Die Peristaltikpumpe kann auch als Schlauchrollenpumpe bezeichnet werden. Unter der Rotorachse kann ein physisch vorhandenes Achs- oder Wellenelement oder eine im geometrischen Sinne gedachte Achse verstanden werden. Der Rotor kann auch als Läufer bezeichnet werden. Die Stützfläche kann auch als Lauffläche bezeichnet werden. Das Schlauchsegment ist nicht Bestandteil der Peristaltikpumpe.

In Ausgestaltung der Erfindung ist das erste Betätigungselement linearbeweglich zwischen der ersten Betätigungsstellung und der zweiten Betätigungsstellung geführt. Eine solche linearbewegliche Führung ist konstruktiv besonders einfach und bauraumsparend umsetzbar. Vorzugsweise ist das Betätigungselement entlang einer quer, besonders bevorzugt senkrecht, zur Rotorachse orientierten Führungsrichtung linearbeweglich geführt. Zur linearbeweglichen Führung des ersten Betätigungselements weist der Rotor vorzugsweise eine entlang einer Führungsachse längserstreckten Linearführung auf. Diese kann zwischen dem Rotorgrundkörper und dem ersten Betätigungselement und/oder zwischen dem ersten Betätigungselement und einem fest mit dem Rotorgrundkörper verbundenen Element ausgebildet sein. Bei dieser Ausgestaltung der Erfindung kann die erste Betätigungsstellung auch als radial eingeschobene oder eingezogene Betätigungsstellung bezeichnet werden. Dementsprechend kann die zweite Betätigungsstellung auch als radial ausgezogene oder ausgeschobene Betätigungsstellung bezeichnet werden.

In weiterer Ausgestaltung der Erfindung ist das erste Betätigungselement an einer Oberseite des Rotorgrundkörpers angeordnet und in einer parallel zu einer Rotationsebene des Rotors erstreckten Führungsebene zwischen der ersten Betätigungsstellung und der zweiten Betätigungsstellung beweglich. Dies gewährleistet zum einen eine ergonomische manuelle Zugänglichkeit des ersten Betätigungselements und/oder des ersten Betätigungsabschnitts sowohl in der ersten als auch in der zweiten Betätigungsstellung. Zudem wird hierdurch eine besonders bauraumsparende Anordnung erreicht. Die Rotationsebene des Rotors ist senkrecht zur Rotorachse erstreckt. Vorzugsweise ist das erste Betätigungselement innerhalb der Führungsebene linearbeweglich. Weiter vorzugsweise ist das erste Betätigungselement entlang einer innerhalb der Führungsebene erstreckten Führungsachse beweglich.

In weiterer Ausgestaltung der Erfindung weist der Rotor ein zweites Betätigungselement mit einem zweiten Betätigungsabschnitt auf, wobei der erste Betätigungsabschnitt und der zweite Betätigungsabschnitt radial entgegengesetzt von der Rotorachse beabstandet angeordnet sind. Das zweite Betätigungselement ist zur manuellen Drehbetätigung des Rotors um die Rotorachse eingerichtet. Der zweite Betätigungsabschnitt ist zum Aufbringen einer weiteren manuellen Drehbetätigungskraft, die zusätzlich zu der an dem ersten Betätigungsabschnitt aufbringbaren manuellen Drehbetätigungskraft wirken kann, eingerichtet. Der zweite Betätigungsabschnitt ist radial entgegengesetzt zu dem ersten Betätigungsabschnitt um eine Hebelarmlänge von der Rotorachse beabstandet. Das zweite Betätigungselement ist wenigstens mittelbar an dem Rotorgrundkörper gelagert und kann relativ zu demselben beweglich oder feststehend sein. Diese Ausgestaltung der Erfindung bietet insbesondere ergonomische Vorteile. Vorzugsweise sind das erste Betätigungselement und das zweite Betätigungselement unter Ausbildung einer Betätigungsanordnung an dem Rotorgrundkörper, bevorzugt an dessen Oberseite, angeordnet, wobei die Betätigungsanordnung zur Drehbetätigung zwischen den Fingern und dem Daumen einer Hand greifbar ist.

In weiterer Ausgestaltung der Erfindung sind das erste Betätigungselement und das zweite Betätigungselement symmetrisch zueinander angeordnet. Vorzugsweise ist eine spiegel- und/oder punktsymmetrische Anordnung vorgesehen. Die symmetrische Anordnung ist vorzugsweise in Bezug auf eine radial erstreckte Symmetrieebene des Rotors und/oder des Rotorgrundkörpers ausgerichtet. Die symmetrische, vorzugsweise spiegelsymmetrische, Anordnung der beiden Betätigungselemente ist vorzugsweise lediglich in einer der beiden Betätigungsstellungen des ersten Betätigungselements gegeben. Diese Ausgestaltung der Erfindung ermöglicht insbesondere eine nochmals verbesserte Ergonomie.

In weiterer Ausgestaltung der Erfindung ist das zweite Betätigungselement relativ zu dem Rotorgrundkörper feststehend an demselben angeordnet und/oder ausgebildet. Vorzugsweise ist das zweite Betätigungselement an der Oberseite des Rotorgrundkörpers angeordnet und/oder ausgebildet. Im Vergleich zu einer alternativen Ausgestaltung, bei welcher sowohl das erste als auch das zweite Betätigungselement relativ zu dem Rotorgrundkörper beweglich an demselben gelagert sind, bietet diese Ausgestaltung der Erfindung einen vereinfachten Aufbau. Das zweite Betätigungselement kann einstückig am Grundkörper ausgebildet oder mittels einer Fügeverbindung mit dem Rotorgrundkörper zusammengefügt sein.

In weiterer Ausgestaltung der Erfindung ist eine baulich von dem Rotorgrundkörper getrennt zwischen dem ersten Betätigungselement und dem zweiten Betätigungselement ausgebildete Linearführung vorgesehen, mittels derer das erste Betätigungselement zwischen der ersten Betätigungsstellung und der zweiten Betätigungsstellung geführt ist. Die bauliche Trennung der Linearführung von dem Rotorgrundkörper bietet insbesondere konstruktive Vorteile. Beispielsweise kann auf eine konstruktive Anpassung des Rotorgrundkörpers zum Zwecke der beweglichen Lagerung des ersten Betätigungselements verzichtet werden. Die Linearführung ist vorzugsweise entlang einer radial zu der Rotorachse orientierten Führungsrichtung und/oder -achse längserstreckt. Weiter vorzugsweise weist die Linearführung eine erste Führungsprofilierung und eine zweite Führungsprofilierung auf, die entlang der Führungsrichtung gleitbeweglich und senkrecht zur Führungsrichtung formschlüssig zusammenwirken. Die erste Führungsprofilierung ist vorzugsweise an dem ersten Betätigungselement ausgebildet. Die zweite Führungsprofilierung ist vorzugsweise an dem zweiten Betätigungselement ausgebildet. Bei einer Ausgestaltung der Erfindung ist die Linearführung in Form einer Schwalbenschwanzführung gestaltet. Das erste Betätigungselement ist entlang der Linearführung begrenzt zwischen der ersten und der zweiten Betätigungsstellung verlagerbar. Zur Begrenzung der Verlagerbarkeit des ersten Betätigungselements sind vorzugsweise an dem ersten und/oder zweiten Betätigungselement ausgebildete Anschlagabschnitte vorgesehen.

In weiterer Ausgestaltung der Erfindung ist das zweite Betätigungselement wenigstens mittelbar an dem Rotorgrundkörper gelagert und relativ zu dem Rotorgrundkörper radial entgegengesetzt zu dem ersten Betätigungselement zwischen unterschiedlichen Betätigungsstellungen beweglich. Vorzugsweise ist das zweite Betätigungselement linearbeweglich zwischen den unterschiedlichen Betätigungsstellungen geführt. Weiter vorzugsweise ist das zweite Betätigungselement gemeinsam mit dem ersten Betätigungselement an einer Oberseite des Rotorgrundkörpers angeordnet und in einer parallel zu einer Rotationsebene des Rotors erstreckten Führungsebene zwischen den unterschiedlichen Betätigungsstellungen beweglich. In einer ersten Betätigungsstellung des zweiten Betätigungselements ist der zweite Betätigungsabschnitt in Bezug auf die Rotorachse radial innenliegend angeordnet. In einer zweiten Betätigungsstellung des zweiten Betätigungselements ist der zweite Betätigungsabschnitt in Bezug auf die Rotorachse radial außenliegend angeordnet. In den unterschiedlichen Betätigungsstellungen des zweiten Betätigungselements wirken demnach entsprechend unterschiedliche Hebelarmlängen. Hierdurch kann bei gleichbleibender Drehbetätigungskraft bedarfsweise ein nochmals erhöhtes manuelles Antriebsdrehmoment erreicht werden. Bei dieser Ausgestaltung der Erfindung sind das erste Betätigungselement und das zweite Betätigungselement vorzugsweise mittels einer an dem Rotorgrundkörper angeordneten Linearführung zwischen ihren unterschiedlichen Betätigungsstellungen geführt.

Die Erfindung betrifft zudem einen Rotor für eine Peristaltikpumpe gemäß der vorhergehenden Beschreibung, aufweisend einen Rotorgrundkörper und wenigstens ein erstes Betätigungselement, welches zur manuellen Drehbetätigung des Rotors um eine Rotorachse eingerichtet ist, und welches wenigstens einen ersten Betätigungsabschnitt zum Aufbringen einer manuellen Drehbetätigungskraft aufweist. Erfindungsgemäß ist das erste Betätigungselement wenigstens mittelbar an dem Rotorgrundkörper gelagert und relativ zu dem Rotorgrundkörper wenigstens abschnittsweise beweglich zwischen einer ersten Betätigungsstellung, in welcher der erste Betätigungsabschnitt um eine erste Hebelarmlänge von der Rotorachse beabstandet ist, und einer zweiten Betätigungsstellung, in welcher der erste Betätigungsabschnitt um eine zweite Hebelarmlänge von der Rotorachse beabstandet ist. Zu mit der erfindungsgemäßen Gestaltung des Rotors einhergehenden Vorteilen wird auf die Erläuterungen zu der erfindungsgemäßen Peristaltikpumpe verwiesen. Zu vorteilhaften Ausgestaltungen des erfindungsgemäßen Rotors wird zur Vermeidung von Wiederholungen auf die Beschreibung der Ausgestaltungen der erfindungsgemäßen Peristaltikpumpe und das zu dem dortigen Rotor Gesagte verwiesen.

Die Erfindung betrifft zudem eine Vorrichtung zur extrakorporalen Blutbehandlung mit einer Peristaltikpumpe gemäß der vorhergehenden Beschreibung.

Weitere Vorteile und Merkmale der Erfindung ergeben sich aus den Ansprüchen sowie aus der nachfolgenden Beschreibung bevorzugter Ausführungsbeispiele der Erfindung, die anhand der Zeichnungen dargestellt sind.
- Fig. 1: zeigt eine schematisch stark vereinfachte Darstellung eines Ausschnitts einer Ausführungsform einer erfindungsgemäßen Vorrichtung zur extrakorporalen Blutbehandlung, die mit einer Ausführungsform einer erfindungsgemäßen Peristaltikpumpe versehen ist,
- Fig. 2: eine perspektivische Detaildarstellung eines Rotors der Peristaltikpumpe, wobei ein Betätigungselement eine erste Betätigungsstellung einnimmt,
- Fig. 3: eine weitere schematische Perspektivdarstellung des Rotors nach Fig. 2, wobei das Betätigungselement eine zweite Betätigungsstellung einnimmt,

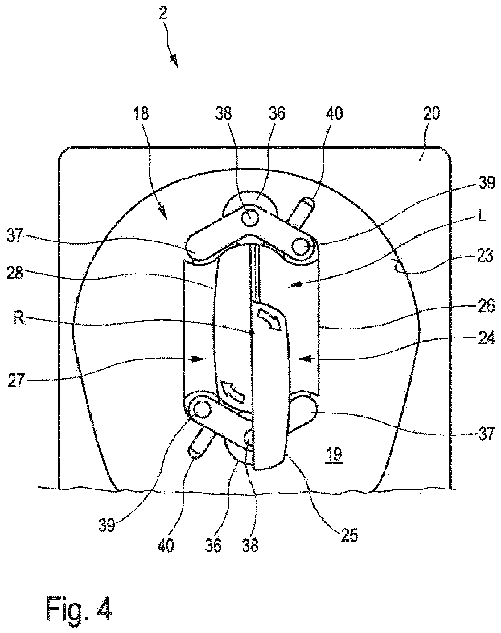

- Fig. 4: in teilweise abgeschnittener Detaildarstellung die Peristaltikpumpe nach Fig. 2 in einer entlang einer Rotorachse gerichteten Blickrichtung und
- Fig. 5: eine weitere Ausführungsform eines erfindungsgemäßen Rotors in einer der Fig. 3 entsprechenden Darstellungsweise.

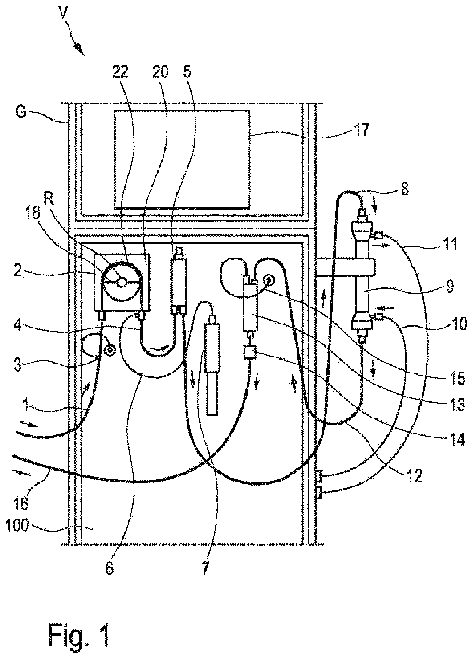

Gemäß Fig. 1 ist eine medizinische Vorrichtung V zur extrakorporalen Blutbehandlung abschnittsweise gezeigt und in Form eines Dialysegeräts gestaltet. Fig. 1 zeigt im Wesentlichen einen gesamten extrakorporalen Blutkreislauf der medizinischen Vorrichtung V. Dieser weist eine arterielle Blutleitung 1 auf, mittels derer zu behandelndes Blut von einem nicht gezeigten Patienten zu einer Peristaltikpumpe 2 der medizinischen Vorrichtung V geführt ist. In Bezug auf eine Förderrichtung des Bluts vor der Peristaltikpumpe 2 ist ein arterieller Druckabnehmer 3 vorgesehen. Mittels des arteriellen Druckabnehmers 3 ist der Druck in der arteriellen Blutleitung 1 vor der Peristaltikpumpe 2 erfassbar. Dieser Druck kann auch als niederdruckseitiger Druck bezeichnet werden. In Förderrichtung des Bluts nach der Peristaltikpumpe 2 - und somit hochdruckseitig - führt eine Hochdruckblutleitung 4 zu einer arteriellen Luftfalle 5. An einem Auslass der Peristaltikpumpe 2 ist vorliegend eine Zuleitung 6 angeordnet, die an einer Pumpe 7 angeschlossen ist. Über die Zuleitung 6 kann Zusatzstoff, beispielsweise Heparin zur Blutverdünnung, hinzudosiert werden. Ausgehend von der arteriellen Luftfalle 5 führt eine Leitung 8 das zu behandelnde Blut zu einem Dialysator 9, welchem eingangsseitig Dialysierflüssigkeit über eine Dialysierflüssigkeitszuleitung 10 zugeführt ist. In dem Dialysator 9 wird das Blut in einer bekannten Weise mittels der Dialysierflüssigkeit behandelt. Verbrauchte Dialysierflüssigkeit, die auch als Dialysat bezeichnet werden kann, wird über eine Dialysierflüssigkeitsableitung 11 von dem Dialysator 9 abgeleitet und einer nicht dargestellten Entsorgung oder Aufbereitung zugeführt. Das zu behandelnde Blut wird mittels einer Blutableitung 12 von dem Dialysator 9 zu einer venösen Luftfalle 13 zur Abscheidung von Luft geleitet. Dieser nachgeschaltet ist ein Luftdetektor/Luftblasendetektor 14, der erkennt, ob sich für den Patienten gefährliche Luft in dem System befindet. An der venösen Luftfalle 13 ist ein venöser Druckabnehmer 15 vorgesehen, mittels dessen der venöse Druck erfassbar ist. Von der Luftfalle 13 über den Luftdetektor/Luftblasendetektor 14 wird das behandelte Blut über eine venöse Blutleitung 16 zurück zum Patienten geleitet. Zudem ist eine Steuer- und Überwachungseinrichtung 17 zur Steuerung und Überwachung der medizinischen Vorrichtung V vorgesehen. Die medizinische Vorrichtung V ist in einem Gehäuse G gekapselt, das eine Gehäusefront 100 aufweist, auf welcher insbesondere die Peristaltikpumpe 2 montiert ist.

Die Peristaltikpumpe 2 weist einen anhand Fig. 1 lediglich stark vereinfacht dargestellten Rotor 18 und ein Pumpengehäuse 20 auf. In einem betriebsfertig montierten Zustand kann das Pumpengehäuse 20 mittels eines zeichnerisch nicht näher dargestellten Deckels abgedeckt sein, der beispielsweise mittels einer ebenfalls nicht näher gezeigten Schwenklageranordnung schwenkbeweglich an dem Pumpengehäuse 20 gelagert ist.

Das Pumpengehäuse 20 weist eine Aufnahmeaussparung 19 auf, in welcher der Rotor 18 um die Rotorachse R rotierbar aufgenommen ist. Zudem weist das Pumpengehäuse 20 eine bogenförmig um die Rotorachse R erstreckte und radial von dem Rotor 18 beabstandete Stützfläche 23 auf. Diese ist zum Abstützen eines radial zwischen den Rotor 18 und die Stützfläche 23 bringbaren Schlauchsegments 22 eingerichtet (Fig. 1). In dem anhand Fig. 1 gezeigten betriebsfertigen Zustand ist das Schlauchsegment 22 in die Aufnahmeaussparung 19 eingebracht und an der Stützfläche 23 abgestützt. An ihren gegenüberliegenden Stirnenden ist das Schlauchsegment 22 auf eine dem Fachmann bekannte Weise mit der arteriellen Blutleitung 1 und der Hochdruckblutleitung 4 fluidleitend verbunden. Zur Pumpförderung des Bluts wirkt der Rotor 18 auf grundsätzlich bekannte Weise auf das Schlauchsegment 22 ein, so dass dieses abschnittsweise elastisch zwischen dem Rotor 18 und der Stützfläche 23 zusammengequetscht wird. Die hierbei entstehende Abquetschung des Schlauchsegments 22, die auch als Okklusion bezeichnet werden kann, wandert gleichsam mit dem rotierenden Rotor 18 um die Rotorachse R, wodurch das Blut von der Niederdruck- auf die Hochdruckseite gefördert wird. Zum Antreiben des Rotors 18 um die Rotorachse R ist ein zeichnerisch nicht näher dargestellter Antriebsmotor vorgesehen. Der Antriebsmotor kann der Peristaltikpumpe 2 und/oder der medizinischen Vorrichtung V zugeordnet sein und ist um die Rotorachse R drehmomentübertragend mit dem Rotor 18 wirkverbunden.

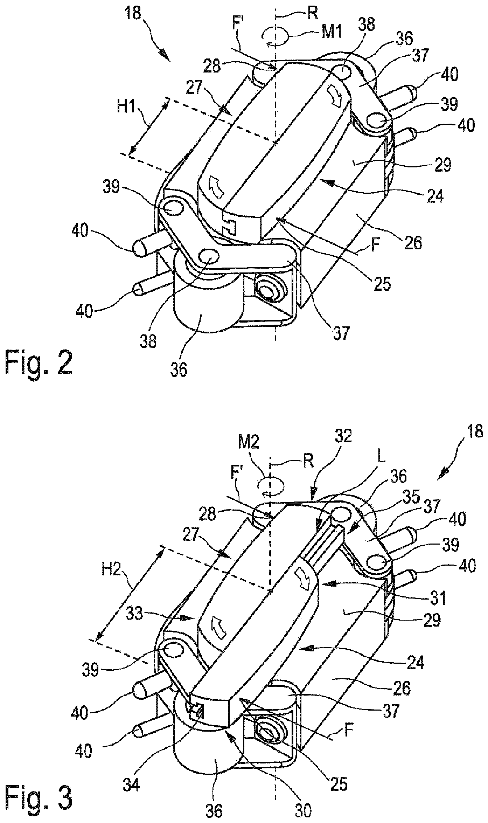

Insbesondere bei einer Beeinträchtigung des Antriebsmotors oder bei einem Einlegen und/oder Herausnehmen des Schlauchsegments 22 zur betriebsfertigen Vorbereitung der medizinischen Vorrichtung V kann ein manueller Antrieb des Rotors 18 erforderlich und/oder wünschenswert sein. Zu diesem Zweck weist der Rotor 18 wenigstens ein erstes Betätigungselement 24 auf, das zur manuellen Drehbetätigung des Rotors 18 um die Rotorachse R eingerichtet ist. Das erste Betätigungselement 24 weist wenigstens einen ersten Betätigungsabschnitt 25 auf, der zum Aufbringen einer manuellen Drehbetätigungskraft vorgesehen ist. Wie insbesondere anhand der Fig. 2 und 3 gezeigt ist, ist das erste Betätigungselement 24 wenigstens mittelbar an einem Rotorgrundkörper 26 gelagert und relativ zu demselben wenigstens abschnittsweise zwischen unterschiedlichen Betätigungsstellungen, nämlich einer ersten Betätigungsstellung (Fig. 2) und einer zweiten Betätigungsstellung (Fig. 3), beweglich. In der ersten Betätigungsstellung ist der Betätigungsabschnitt 25 um eine erste Hebelarmlänge H1 von der Rotorachse R beabstandet. In der zweiten Betätigungsstellung ist der erste Betätigungsabschnitt 25 um eine zweite Hebelarmlänge H2 von der Rotorachse R beabstandet.

Dabei ist die zweite Hebelarmlänge H2 größer als die erste Hebelarmlänge H1. Hierdurch bewirkt eine an dem ersten Betätigungsabschnitt 25 angreifende manuelle Drehbetätigungskraft F je nach Betätigungsstellung des ersten Betätigungselements 24 unterschiedliche um die Rotorachse R wirksame manuelle Antriebsdrehmomente M1, M2. Dabei ist das in der zweiten Betätigungsstellung wirksame manuelle Antriebsdrehmoment M2 proportional zum Verhältnis der Hebelarmlängen H1, H2 größer als das in der ersten Betätigungsstellung wirksame manuelle Antriebsdrehmoment M1. Vereinfacht ausgedrückt, ist der Rotor 18 in der zweiten Betätigungsstellung manuell leichtgängiger drehbar als in der ersten Betätigungsstellung. Der erste Betätigungsabschnitt 25 ist sowohl in der ersten als auch in der zweiten Betätigungsstellung uneingeschränkt manuell zugänglich.

Bei der gezeigten Ausführungsform weist der Rotor 18 ein zweites Betätigungselement 27 mit einem zweiten Betätigungsabschnitt 28 auf. Der zweite Betätigungsabschnitt 28 ist radial entgegengesetzt von dem ersten Betätigungsabschnitt 24 beabstandet angeordnet.

Zur manuellen Drehbetätigung des Rotors 18 werden das erste Betätigungselement 24 und das zweite Betätigungselement 27 zwischen dem Daumen und den Fingern einer Hand gegriffen und unter Einwirkung der manuellen Drehbetätigungskraft F und einer weiteren Drehbetätigungskraft F` um die Rotorachse R gedreht. Dabei wirkt die Drehbetätigungskraft F an dem ersten Betätigungsabschnitt 25. Die weitere Drehbetätigungskraft F` wirkt an dem zweiten Betätigungsabschnitt 28.

Beide Betätigungselemente 24, 27 sind vorliegend an einer Oberseite 29 des Rotorgrundkörpers 26 angeordnet. Zudem sind das erste Betätigungselement 24 und das zweite Betätigungselement 27 - jedenfalls in der ersten Betätigungsstellung - spiegelsymmetrisch in Bezug auf eine nicht näher bezeichnete Mittellängsachse des Rotorgrundkörpers 26 angeordnet.

Bei einer zeichnerisch nicht dargestellten Ausführungsform ist eine punktsymmetrische Anordnung vorgesehen.

Das erste Betätigungselement 24 und das zweite Betätigungselement 27 weisen jeweils eine leisten-, riegel- und/oder quaderförmige Grundform auf. Das erste Betätigungselement 24 ist in radialer Richtung zwischen einem ersten Ende 30 und einem zweiten Ende 31 längserstreckt. Der erste Betätigungsabschnitt 25 ist im Bereich des ersten Endes 30 angeordnet. Das zweite Betätigungselement 27 ist in radialer Richtung zwischen einem ersten Ende 32 und einem zweiten Ende 33 längserstreckt. Der zweite Betätigungsabschnitt 28 ist im Bereich des ersten Endes 32 angeordnet.

In der ersten Betätigungsstellung des ersten Betätigungselements 24 (Fig. 2) ist der zweite Betätigungsabschnitt 28 um eine zeichnerisch nicht näher dargestellte Hebelarmlänge von der Rotorachse R beabstandet, die betragsmäßig mit der ersten Hebelarmlänge H1 übereinstimmt. In der ersten Betätigungsstellung bilden das erste Betätigungselement 24 und das zweite Betätigungselement 27 eine von der Oberseite 29 entlang der Rotorachse R aufragende Betätigungsanordnung in Form eines Drehknebels aus, wobei jeweils eines der Betätigungselemente 24, 27 eine Hälfte der besagten Anordnung bzw. des besagten Drehknebels bildet. In der ersten Betätigungsstellung sind das erste Ende 30 des ersten Betätigungselements 24 und das zweite Ende 33 des zweiten Betätigungselements in radialer Richtung zueinander fluchtend ausgerichtet. Entsprechendes gilt in Bezug auf das zweite Ende 31 des ersten Betätigungselements 24 und das erste Ende 32 des zweiten Betätigungselements 27.

Bei der gezeigten Ausführungsform ist das erste Betätigungselement 24 relativ zu dem Rotorgrundkörper 26 und/oder dem zweiten Betätigungselement 27 linearbeweglich zwischen der ersten Betätigungsstellung (Fig. 2) und der zweiten Betätigungsstellung (Fig. 3) geführt. Zu diesem Zweck weist der Rotor 18 eine noch näher beschriebene Linearführung L auf.

Bei zeichnerisch nicht näher dargestellten Ausführungsformen kann das erste Betätigungselement stattdessen schwenk- oder drehbeweglich zwischen der ersten Betätigungsstellung und der zweiten Betätigungsstellung geführt sein. Zu diesem Zweck kann der Rotor eine Schwenk- und/oder Drehführung aufweisen.

Bei der gezeigten Ausführungsform ist das erste Betätigungselement 24 zwischen der ersten und der zweiten Betätigungsstellung starrkörperbeweglich. Dementsprechend wird nicht lediglich der erste Betätigungsabschnitt 25, sondern stattdessen das gesamte erste Betätigungselement 24 zwischen den Betätigungsstellungen verlagert. Bei einer nicht gezeigten Ausführungsform kann das erste Betätigungselement stattdessen beispielsweise mit einer Teleskopführung oder dergleichen versehen sein, die eine linearbewegliche Verlagerung lediglich des ersten Betätigungsabschnitts 25 relativ zu dem Rotorgrundkörper 26 gestattet.

Die linearbewegliche Führung des ersten Betätigungselements 24 erfolgt in einer parallel zu einer Rotationsebene des Rotors 18 erstreckten Führungsebene, die bei der gezeigten Ausführungsform parallel zu der Oberseite 29 ausgerichtet ist und/oder mit derselben zusammenfällt.

In der zweiten Betätigungsstellung ist der erste Betätigungsabschnitt 25 und damit auch das erste Ende 30 in radialer Richtung nach außen verlagert, so dass auch von einer ausgezogenen oder ausgeschobenen (zweiten) Betätigungsstellung gesprochen werden kann. Dementgegen ist der erste Betätigungsabschnitt 25 und damit auch das erste Ende 30 in der ersten Betätigungsstellung - relativ zu der zweiten Betätigungsstellung - in radialer Richtung nach innen verlagert, so dass auch von einer eingeschobenen oder eingezogenen (ersten) Betätigungsstellung gesprochen werden kann.

Im Unterschied zu dem ersten Betätigungselement 24 ist das zweite Betätigungselement 27 relativ zu dem Rotorgrundkörper 26 feststehend an demselben angeordnet und/oder ausgebildet. Bei der gezeigten Ausführungsform ist das zweite Betätigungselement 27 separat von dem Rotorgrundkörper 26 gefertigt und hiernach auf eine dem Fachmann bekannte Weise fest mit dessen Oberseite 29 zusammengefügt. Bei einer nicht gezeigten Ausführungsform ist das zweite Betätigungselement 27 stattdessen einstückig mit dem Rotorgrundkörper 26 ausgebildet.

Zur linearbeweglichen Führung des ersten Betätigungselements 24 weist der Rotor 18 die Linearführung L auf. Bei der gezeigten Ausführungsform ist die Linearführung L baulich von dem Rotorgrundkörper 26 getrennt zwischen dem ersten Betätigungselement 24 und dem zweiten Betätigungselement 27 ausgebildet. Die Linearführung L weist eine an dem ersten Betätigungselement 24 ausgebildete erste Führungsprofilierung 34 und eine an dem zweiten Betätigungselement 27 ausgebildete zweite Führungsprofilierung 35 auf. Die erste Führungsprofilierung 34 und die zweite Führungsprofilierung 35 wirken in radialer Richtung der Rotorachse R gleitbeweglich und senkrecht hierzu formschlüssig zusammen. Die erste Führungsprofilierung 34 ist in Form einer T-förmigen Führungsnut gestaltet. Die zweite Führungsprofilierung 35 ist hierzu komplementär in Form einer T-förmigen Führungsleiste gestaltet.

Die Linearführung L weist zudem zeichnerisch nicht näher dargestellte Anschlagsabschnitte auf, die gewährleisten, dass das erste Betätigungselement 24 in radialer Richtung unverlierbar an dem zweiten Betätigungselement 27 gehalten ist. Mit anderen Worten ausgedrückt, verhindern die Anschlagabschnitte, dass das erste Betätigungselement 24 in radialer Richtung entlang der Linearführung L von dem zweiten Betätigungselement 27 abziehbar ist.

Im Übrigen weist der Rotorgrundkörper 26 bei der gezeigten Ausführungsform eine quaderförmige Grundform auf. An in radialer Richtung der Rotorachse R gegenüberliegenden Stirnenden des Rotorgrundkörpers 26 weist der Rotor 18 jeweils eine Druckrolle 36 auf, die um eine parallel zu der Rotorachse R erstreckte Achse 38 drehbar an einem Andruckhebel 37 gelagert ist. Die Andruckhebel 37 sind jeweils um eine ebenfalls parallel zur Rotorachse R erstreckte weitere Achse 39 relativ zu dem Rotorgrundkörper 26 schwenkbeweglich an demselben gelagert. Um ein unbeabsichtigtes Herausdrücken des Schlauchsegments 22 zwischen dem Rotor 18 und der Stützfläche 23 zu vermeiden, weist der Rotor 18 zudem Führungsstifte 40 auf. Die Führungsstifte 40 sind in Rotationsrichtung des Rotors 18 den Druckrollen 36 vorgelagert an dem jeweiligen Andruckhebel 37 angeordnet. Die Führungsstifte 40 sind jeweils paarweise und in axialer Richtung beabstandet an dem jeweiligen Andruckhebel 37 angeordnet, so dass das Schlauchsegment 22 relativ zu dem rotierenden Rotor 18 in axialer Richtung zwischen den axial beabstandeten Führungsstiften 40 geführt ist.

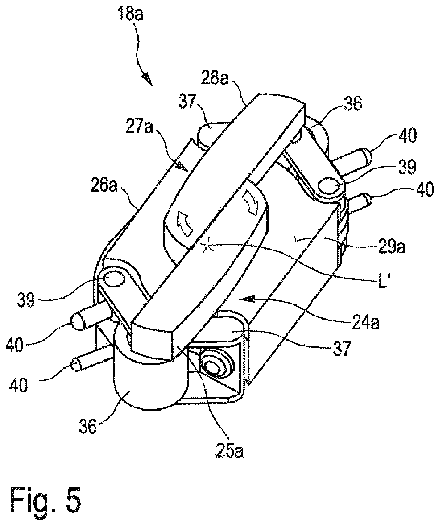

Anhand Fig. 5 ist eine weitere Ausführungsform eines erfindungsgemäßen Rotors 18a gezeigt, der hinsichtlich seiner räumlich-körperlichen und funktionellen Gestaltung im Wesentlichen mit der Ausführungsform nach den Fig. 2 und 3 übereinstimmt. Zur Vermeidung von Wiederholungen wird nachfolgend lediglich auf wesentliche Unterschiede des Rotors 18a nach Fig. 5 gegenüber dem Rotor 18 nach den Fig. 2 und 3 eingegangen, wobei identische Bauteile und/oder Abschnitte mit identischen Bezugszeichen versehen sind. Unterschiedlich gestaltete Bauteile und/oder Abschnitte sind unter Hinzufügung des Kleinbuchstabens a zu der Bezugszeichenziffer gekennzeichnet. Identische Bauteile und/oder Abschnitte werden nicht gesondert erläutert. Stattdessen wird auf die diesbezügliche Offenbarung in Zusammenhang mit dem Rotor 18 verwiesen, die sinngemäß auch im Hinblick auf den Rotor 18a gilt.

Im Unterschied zu der Ausführungsform nach den Fig. 2 und 3 sind bei dem Rotor 18a sowohl das erste Betätigungselement 24a als auch das zweite Betätigungselement 27a jeweils zwischen unterschiedlichen Betätigungsstellungen relativ zu dem Rotorgrundkörper 26a linearbeweglich verlagerbar. Anhand Fig. 5 ist eine Konfiguration gezeigt, in welcher beide Betätigungselemente 24a, 27a ihre jeweils radial ausgezogene bzw. ausgeschobene Betätigungsstellung einnehmen. Zu diesem Zweck sind beide Betätigungselemente 24a, 27a linearbeweglich an dem Rotorgrundkörper 26a geführt. Zu diesem Zweck ist eine zeichnerisch nicht näher ersichtliche Linearführung L' vorgesehen, die zwischen dem Rotorgrundkörper 26a und den beiden Betätigungselementen 24a, 27a ausgebildet ist. Die Linearführung L' weist komplementäre Führungsprofilierungen auf, die eine in radialer Richtung gleitbewegliche Linearführung und senkrecht hierzu formschlüssige Lagerung der beiden Betätigungselemente 24a, 27a an dem Rotorgrundkörper 26a gewährleisten. Die komplementären Führungsprofilierungen sind zum einen an der Oberseite 29a des Rotorgrundkörpers 26a und zum anderen unterseitig an den beiden Betätigungselementen 24a, 27a angeordnet und/oder ausgebildet. Alternativ oder zusätzlich können die Führungsprofilierungen jeweils als mittiger Steg ausgebildet sein.

In der eingeschobenen bzw. eingezogenen Stellung der beiden Betätigungselemente 24a, 27a ergibt sich eine Konfiguration, die hinsichtlich der resultierenden Hebelarmlängen und fluchtenden Anordnung der Betätigungselemente 24a, 27a identisch mit der anhand Fig. 2 ersichtlichen Konfiguration ist.

## Patentansprüche

1. Peristaltikpumpe (2) für eine Vorrichtung (V) zur extrakorporalen Blutbehandlung, aufweisend
- einen um eine Rotorachse (R) rotierbaren Rotor (18, 18a) mit einem Rotorgrundkörper (26, 26a) und
- ein Pumpengehäuse (20) mit einer Aufnahmeaussparung (19), in welcher der Rotor (18, 18a) aufgenommen ist, und welche eine bogenförmig um die Rotorachse (R) erstreckte und radial von dem Rotor (18, 18a) beabstandete Stützfläche (23) aufweist, die zum Abstützen eines radial zwischen den Rotor (18, 18) und die Stützfläche (23) einbringbaren Schlauchsegments (22) eingerichtet ist,
- wobei der Rotor (18, 18a) wenigstens ein erstes Betätigungselement (24, 24a) aufweist, welches zur manuellen Drehbetätigung des Rotors (18, 18a) um die Rotorachse (R) eingerichtet ist, und welches wenigstens einen ersten Betätigungsabschnitt (25, 25a) zum Aufbringen einer manuellen Drehbetätigungskraft (F) aufweist,
- **dadurch gekennzeichnet, dass** das erste Betätigungselement (24, 24a) wenigstens mittelbar an dem Rotorgrundkörper (26, 26a) gelagert und relativ zu dem Rotorgrundkörper (26, 26a) wenigstens abschnittsweise beweglich ist zwischen einer ersten Betätigungsstellung, in welcher der erste Betätigungsabschnitt (25a) um eine erste Hebelarmlänge (H1) von der Rotorachse (R) beabstandet ist, und einer zweiten Betätigungsstellung, in welcher der erste Betätigungsabschnitt (25a) um eine zweite Hebelarmlänge (H2) von der Rotorachse (R) beabstandet ist, wobei die zweite Hebelarmlänge (H2) größer ist als die erste Hebelarmlänge (H1).

2. Peristaltikpumpe (2) nach Anspruch 1, **dadurch gekennzeichnet, dass** das erste Betätigungselement (24, 24a) linearbeweglich zwischen der ersten Betätigungsstellung und der zweiten Betätigungsstellung geführt ist.

3. Peristaltikpumpe (2) nach Anspruch 1 oder 2, **dadurch gekennzeichnet, dass** das erste Betätigungselement (24, 24a) an einer Oberseite (29, 29a) des Rotorgrundkörpers (26, 26a) angeordnet und in einer parallel zu einer Rotationsebene des Rotors (18, 18a) erstreckten Führungsebene zwischen der ersten Betätigungsstellung und der zweiten Betätigungsstellung beweglich ist.

4. Peristaltikpumpe (2) nach einem der vorhergehenden Ansprüche, **dadurch gekennzeichnet, dass** der Rotor (18, 18a) ein zweites Betätigungselement (27, 27a) mit einem zweiten Betätigungsabschnitt (28, 28a) aufweist, wobei der erste Betätigungsabschnitt (25, 25a) und der zweite Betätigungsabschnitt (28, 28a) radial entgegengesetzt von der Rotorachse (R) beabstandet angeordnet sind.

5. Peristaltikpumpe (2) nach Anspruch 4, **dadurch gekennzeichnet, dass** das erste Betätigungselement (24, 24a) und das zweite Betätigungselement (27, 27a) symmetrisch zueinander angeordnet sind.

6. Peristaltikpumpe (2) nach einem der Ansprüche 4 oder 5, **dadurch gekennzeichnet, dass** das zweite Betätigungselement (27) relativ zu dem Rotorgrundkörper (26) feststehend an demselben angeordnet und/oder ausgebildet ist.

7. Peristaltikpumpe (2) nach Anspruch 6, **dadurch gekennzeichnet, dass** eine baulich von dem Rotorgrundkörper (26) getrennt zwischen dem ersten Betätigungselement (24) und dem zweiten Betätigungselement (27) ausgebildete Linearführung (L) vorgesehen ist, mittels derer das erste Betätigungselement (24) zwischen der ersten Betätigungsstellung und der zweiten Betätigungsstellung geführt ist.

8. Peristaltikpumpe (2) nach Anspruch 4 oder 5, **dadurch gekennzeichnet, dass** das zweite Betätigungselement (27a) wenigstens mittelbar an dem Rotorgrundkörper (26a) gelagert und relativ zu dem Rotorgrundkörper (26a) radial entgegengesetzt zu dem ersten Betätigungselement (24a) zwischen unterschiedlichen Betätigungsstellungen beweglich ist.

9. Rotor (18, 18a) für eine Peristaltikpumpe (2) nach einem der vorhergehenden Ansprüche, aufweisend einen Rotorgrundkörper (26, 26a) und wenigstens ein erstes Betätigungselement (24, 24a), welches zur manuellen Drehbetätigung des Rotors (18, 18a) um eine Rotorachse (R) eingerichtet ist, und welches wenigstens einen ersten Betätigungsabschnitt (25, 25a) zum Aufbringen einer manuellen Drehbetätigungskraft (F) aufweist, **dadurch gekennzeichnet, dass** das erste Betätigungselement (24, 24a) wenigstens mittelbar an dem Rotorgrundkörper (26, 26a) gelagert und relativ zu dem Rotorgrundkörper (26, 26a) wenigstens abschnittsweise beweglich ist zwischen einer ersten Betätigungsstellung, in welcher der erste Betätigungsabschnitt (25, 25a) um eine erste Hebelarmlänge (H1) von der Rotorachse (R) beabstandet ist, und einer zweiten Betätigungsstellung, in welcher der erste Betätigungsabschnitt (25, 25a) um eine zweite Hebelarmlänge (H2) von der Rotorachse (R) beabstandet ist.

10. Vorrichtung (V) zur extrakorporalen Blutbehandlung mit einer Peristaltikpumpe (2) nach einem der Ansprüche 1 bis 8.

## Claims

1. A peristaltic pump (2) for a device (V) for extracorporeal blood treatment, having
- a rotor (18, 18a), which can be rotated about a rotor axis (R) and has a main rotor body (26, 26a), and
- a pump housing (20) having a receiving recess (19), in which the rotor (18, 18a) is received and which has a support surface (23) that extends arcuately around the rotor axis (R), is spaced apart radially from the rotor (18, 18a) and is configured to support a hose segment (22) that can be introduced radially between the rotor (18, 18a) and the support surface (23),
- wherein the rotor (18, 18a) has at least one first actuation element (24, 24a), which is configured for manual rotary actuation of the rotor (18, 18a) about the rotor axis (R) and which has at least one first actuation section (25, 25a) for applying a manual rotary actuation force (F),
- **characterized in that** the first actuation element (24, 24a) is mounted at least indirectly on the main rotor body (26, 26a) and at least some section or sections thereof can be moved relative to the main rotor body (26, 26a) between a first actuation position, in which the first actuation section (25a) is spaced apart from the rotor axis (R) by a first lever arm length (H1), and a second actuation position, in which the first actuation section (25a) is spaced apart from the rotor axis (R) by a second lever arm length (H2), wherein the second lever arm length (H2) is greater than the first lever arm length (H1).

2. The peristaltic pump (2) as claimed in claim 1, **characterized in that** the first actuation element (24, 24a) is guided in a linearly movable manner between the first actuation position and the second actuation position.

3. The peristaltic pump (2) as claimed in claim 1 or 2, **characterized in that** the first actuation element (24, 24a) is arranged on an upper side (29, 29a) of the main rotor body (26, 26a) and can be moved between the first actuation position and the second actuation position in a guidance plane extending parallel to a plane of rotation of the rotor (18, 18a) .

4. The peristaltic pump (2) as claimed in any of the preceding claims, **characterized in that** the rotor (18, 18a) has a second actuation element (27, 27a) with a second actuation section (28, 28a), wherein the first actuation section (25, 25a) and the second actuation section (28, 28a) are arranged radially opposite at a distance from the rotor axis (R).

5. The peristaltic pump (2) as claimed in claim 4, **characterized in that** the first actuation element (24, 24a) and the second actuation element (27, 27a) are arranged symmetrically with respect to one another.

6. The peristaltic pump (2) as claimed in claim 4 or 5, **characterized in that** the second actuation element (27) is arranged and/or formed on the main rotor body (26) in such a way as to be stationary relative thereto.

7. The peristaltic pump (2) as claimed in claim 6, **characterized in that** a linear guide (L) which is formed in a structurally separate manner from the main rotor body (26) is provided between the first actuation element (24) and the second actuation element (27), by means of which linear guide (L) the first actuation element (24) is guided between the first actuation position and the second actuation position.

8. The peristaltic pump (2) as claimed in claim 4 or 5, **characterized in that** the second actuation element (27a) is mounted at least indirectly on the main rotor body (26a) and can be moved relative to the main rotor body (26a) in a radially opposite manner to the first actuation element (24a) between different actuation positions.

9. A rotor (18, 18a) for a peristaltic pump (2) as claimed in any of the preceding claims, having a main rotor body (26, 26a) and at least one first actuation element (24, 24a), which is configured for manual rotary actuation of the rotor (18, 18a) about a rotor axis (R) and which has at least one first actuation section (25, 25a) for applying a manual rotary actuation force (F), **characterized in that** the first actuation element (24, 24a) is mounted at least indirectly on the main rotor body (26, 26a) and at least some section or sections thereof can be moved relative to the main rotor body (26, 26a) between a first actuation position, in which the first actuation section (25, 25a) is spaced apart from the rotor axis (R) by a first lever arm length (H1), and a second actuation position, in which the first actuation section (25, 25a) is spaced apart from the rotor axis (R) by a second lever arm length (H2) .

10. A device (V) for extracorporeal blood treatment having a peristaltic pump (2) as claimed in any of claims 1 to 8.

## Revendications

1. Pompe péristaltique (2) pour un dispositif (V) de traitement de sang extracorporel, présentant
- un rotor (18, 18a) pouvant tourner autour d'un axe de rotor (R) avec un corps de base de rotor (26, 26a) et
- un boîtier de pompe (20) avec un évidement de réception (19), dans lequel est reçu le rotor (18, 18a), et qui présente une surface de soutien (23) s'étendant en forme d'arc autour de l'axe de rotor (R) et espacée radialement du rotor (18, 18a), qui est adaptée pour soutenir un segment de tuyau (22) pouvant être introduit radialement entre le rotor (18, 18) et la surface de soutien (23),
- le rotor (18, 18a) présentant au moins un premier élément d'actionnement (24, 24a) qui est adapté pour l'actionnement en rotation manuel du rotor (18, 18a) autour de l'axe de rotor (R), et qui présente au moins une première section d'actionnement (25, 25a) pour appliquer une force d'actionnement en rotation manuelle (F),
- **caractérisée en ce que** le premier élément d'actionnement (24, 24a) est monté au moins indirectement sur le corps de base de rotor (26, 26a) et est mobile par rapport au corps de base de rotor (26, 26a) au moins par sections entre une première position d'actionnement, dans laquelle la première section d'actionnement (25a) est espacée de l'axe de rotor (R) d'une première longueur de bras de levier (H1), et une deuxième position d'actionnement dans laquelle la première section d'actionnement (25a) est espacée de l'axe de rotor (R) d'une deuxième longueur de bras de levier (H2), la deuxième longueur de bras de levier (H2) étant supérieure à la première longueur de bras de levier (H1).

2. Pompe péristaltique (2) selon la revendication 1, **caractérisée en ce que** le premier élément d'actionnement (24, 24a) est guidé de manière mobile linéairement entre la première position d'actionnement et la deuxième position d'actionnement.

3. Pompe péristaltique (2) selon la revendication 1 ou 2, **caractérisée en ce que** le premier élément d'actionnement (24, 24a) est agencé sur un côté supérieur (29, 29a) du corps de base de rotor (26, 26a) et est mobile entre la première position d'actionnement et la deuxième position d'actionnement dans un plan de guidage s'étendant parallèlement à un plan de rotation du rotor (18, 18a).

4. Pompe péristaltique (2) selon l'une quelconque des revendications précédentes, **caractérisée en ce que** le rotor (18, 18a) présente un deuxième élément d'actionnement (27, 27a) avec une deuxième section d'actionnement (28, 28a), la première section d'actionnement (25, 25a) et la deuxième section d'actionnement (28, 28a) étant agencées en étant espacées de l'axe de rotor (R) de manière radialement opposée.

5. Pompe péristaltique (2) selon la revendication 4, **caractérisée en ce que** le premier élément d'actionnement (24, 24a) et le deuxième élément d'actionnement (27, 27a) sont agencés symétriquement l'un par rapport à l'autre.

6. Pompe péristaltique (2) selon l'une quelconque des revendications 4 ou 5, **caractérisée en ce que** le deuxième élément d'actionnement (27) est agencé et/ou réalisé sur le corps de base de rotor (26) de manière fixe par rapport à celui-ci.

7. Pompe péristaltique (2) selon la revendication 6, **caractérisée en ce qu'**il est prévu un guide linéaire (L) réalisé de manière séparée par construction du corps de base de rotor (26) entre le premier élément d'actionnement (24) et le deuxième élément d'actionnement (27), au moyen duquel le premier élément d'actionnement (24) est guidé entre la première position d'actionnement et la deuxième position d'actionnement.

8. Pompe péristaltique (2) selon la revendication 4 ou 5, **caractérisée en ce que** le deuxième élément d'actionnement (27a) est monté au moins indirectement sur le corps de base de rotor (26a) et est mobile par rapport au corps de base de rotor (26a), radialement à l'opposé du premier élément d'actionnement (24a), entre différentes positions d'actionnement.

9. Rotor (18, 18a) pour une pompe péristaltique (2) selon l'une quelconque des revendications précédentes, présentant un corps de base de rotor (26, 26a) et au moins un premier élément d'actionnement (24, 24a) qui est adapté pour l'actionnement en rotation manuel du rotor (18, 18a) autour d'un axe de rotor (R), et qui présente au moins une première section d'actionnement (25, 25a) pour appliquer une force d'actionnement en rotation manuelle (F), **caractérisé en ce que** le premier élément d'actionnement (24, 24a) est monté au moins indirectement sur le corps de base de rotor (26, 26a) et est mobile par rapport au corps de base de rotor (26, 26a) au moins par sections entre une première position d'actionnement, dans laquelle la première section d'actionnement (25, 25a) est espacée de l'axe de rotor (R) d'une première longueur de bras de levier (H1), et une deuxième position d'actionnement, dans laquelle la première section d'actionnement (25, 25a) est espacée de l'axe de rotor (R) d'une deuxième longueur de bras de levier (H2).

10. Dispositif (V) de traitement de sang extracorporel, avec une pompe péristaltique (2) selon l'une quelconque des revendications 1 à 8.
